# EUROPÄISCHE PATENTSCHRIFT

(11) **EP 3 020 449 B1**
(45) Veröffentlichungstag und Bekanntmachung des Hinweises auf die Patenterteilung: **21.09.2022**
(21) Anmeldenummer: 15199218.7
(22) Anmeldetag: 11.05.2011
(51) Int. Cl.: A61N 1/375, H01R 13/00

(54) **ELEKTRONISCHES GERÄT BZW. ELEKTRISCHES BAUTEIL**
ELECTRONIC DEVICE OR ELECTRIC COMPONENT
APPAREIL ELECTRONIQUE OU COMPOSANT ELECTRONIQUE

(30) Priorität: 21.05.2010 US 346925 P
(43) Veröffentlichungstag der Anmeldung: 18.05.2016
(62) Teilanmeldung aus: 11165634.4
(73) Patentinhaber: Dyconex AG, 8303 Bassersdorf (CH)
(72) Erfinder: Hauer, Marc, 8610 Uster (CH); Eck, Stefan, 91315 Höchstadt (DE)
(74) Vertreter: Biotronik Corporate Services SE

(56) Entgegenhaltungen:
- EP-A2- 0 776 016
- WO-A2-2009/086435
- US-A1- 2005 040 513
- US-A1- 2007 182 364

## Beschreibung

Die Erfindung betrifft ein elektrisches Bauteil mit einem Gehäuse, einer in dem Gehäuse angeordneten Funktionseinheit, einem die Funktionseinheit nach außen elektrisch anschließenden Anschlussleiter und einer dichten, den Anschlussleiter umgebenden und gegenüber dem Gehäuse isolierenden Durchführung im Gehäuse.

Bei elektronischen Geräten und elektrischen Bauteilen, die besonderen Anforderungen hinsichtlich der dauerhaften Dichtigkeit genügen müssen, sind die sogenannten Durchführungen um einen Anschlussleiter besonders kritische Bereiche. Ihrer Gestaltung wird daher bei solchen Geräten, etwa bei implantierbaren medizin-elektronischen Geräten (Schrittmachern, implantierbaren Kardiovertern, Cochlearimplantaten, implantierbaren Neurostimulatoren etc.) seit Jahren besondere Aufmerksamkeit geschenkt. Technisch etabliert ist dabei die Bildung der Durchführung aus geeigneter Keramik, speziell umgeben von einem metallischen Flansch des Gehäuses.

Speziell für elektrochemische Bauteile, wie etwa Batterien oder Elektrolyt-Kondensatoren, ist aus der WO 2005/001997 A2 eine Durchführung bekannt, bei der ein Führungselement aus einem Polymer zur Aufnahme des Anschlussleiters vorgesehen ist. Aus der US 2007/0225771 A1 ist eine Durchführungsanordnung bekannt, die beispielsweise in einem Stromquellengehäuse eines implantierbaren medizinischen Geräts Anwendung findet und bei der zwischen dem Anschlussleiter und einem (den volumenmäßig größten Bestandteil der Durchführung bildenden) Keramik- oder Glasring eine dünne Hülse vorgesehen ist, die unter anderem zum Toleranzausgleich bei dem thermisch hoch empfindlichen Metall-Keramik-Aufbau der Durchführung dient. Die dünne Hülse kann insbesondere aus Edelstahl, Aluminium oder Titan bestehen.

Aus der US 5,825,608 ist eine Durchführung eines elektronischen Gerätes bekannt, welche zugleich als Filterkondensator wirkt und beispielsweise bei implantierbaren Geräten, wie etwa Herzschrittmachern, Anwendung finden kann. Bei dieser Durchführung wird ein leitfähiges Polymerharz zur Herstellung elektrischer Verbindungen in einem ansonsten im Wesentlichen aus Keramik aufgebauten Durchführungs-Körper eingesetzt.

Aus der WO 2009/086435 A2 sind Durchführungen bekannt, die flüssigkristallinen Polymere enthalten können.

Eine Aufgabe der Erfindung besteht in der Bereitstellung eines verbesserten elektronischen elektrischen Bauteils, welches insbesondere mit hoher Ausbeute hergestellt werden kann, unempfindlich gegenüber thermischen Belastungen ist und einen zuverlässigen Langzeitbetrieb ermöglicht.

Diese Aufgabe wird gemäß einem ersten Aspekt der Erfindung durch ein elektrisches Bauteil mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, die Durchführung des Bauteils aus einem flüssigkristallinen Polymer zu bilden. Hierdurch können insbesondere die bekannten Probleme hinsichtlich der Fertigungstoleranzen und thermischer Belastungen vermieden werden, die bei bekannten Metall-Glas- oder Metall-Keramik-Durchführungskonstruktionen festgestellt wurden.

Die Durchführung ist allein aus dem flüssigkristallinen Polymer gebildet, in dem eine oder mehrere Metallschichten eingelagert und/oder angebracht sind.

In Anbetracht des ungünstigeren Diffusionsverhaltens flüssigkristalliner Polymere, verglichen mit jenem von Keramik, sind des Weiteren Ausführungen von Vorteil, bei denen der Diffusionsquerschnitt weitgehend minimiert und/oder die Diffusionslänge weitgehend maximiert ist. Insofern ist eine weitere Ausführung zweckmäßig, bei der die Durchführung alternierend vom Umfang und von einem Mittenbereich ausgehend verlaufende und jeweils nicht vollständig bis zur Mitte bzw. bis zum Umfang reichende Metallschichten aufweist, die auf den Stirnflächen der Durchführung angeordnet und/oder in dem flüssigkristallinen Polymere innerhalb der Durchführung eingebettet sind und im Längsschnitt eine Labyrinthstruktur bilden. Alternativ hierzu kann vorgesehen sein, dass die Durchführung eine spiralig von nahe der oberen Stirnfläche zu nahe der unteren Stirnfläche verlaufende Metallschicht aufweist. Auch sonstige konstruktive Ausgestaltungen, die dem Ziel eines verringertem Diffusionsquerschnitts bzw. einer erhöhten Diffusionslänge dienen, sind als vorteilhaft anzusehen.

Eine weitere Ausführung der Erfindung sieht vor, dass mindestens eine Metallschicht, insbesondere eine Mehrzahl von Metallschichten einer Labyrinthstruktur, mit dem Anschlussleiter fest verbunden und/oder integral mit diesem gebildet ist.

Das elektrische Bauteil gemäß der Erfindung ist insbesondere als Kondensator ausgebildet oder als Sende- und/oder Baugruppe eine Nachrichtenübertragungs- oder Filteranordnung. Auch sonstige Bauteile, bei denen es auf eine hochgradig dichte und zugleich thermisch stabile Durchführung ankommt, können vorteilhaft den vorgeschlagenen Aufbau aufweisen.

Im Übrigen kann die Durchführung des elektrischen Bauteils auf die gleiche Weise ausgestaltet sein wie einem elektronischen Gerät, das nicht Gegenstand dieser Patentanmeldung ist. Speziell bei den erwähnten konstruktiven Ausgestaltungen, bei denen der LCP mit metallischen Elementen (speziell Schichten) kombiniert ist, lassen sich mit der Durchführung zugleich elektrische Zusatzfunktionen realisieren.

Insbesondere können die metallischen Elemente zusammen mit Abschnitten des flüssigkristallinen Polymers eine Kapazität bilden, welche derart angeordnet und an die Funktionseinheit des Gerätes angeschlossen ist, dass hiermit eine elektrische Funktion, insbesondere eine Filter- bzw. frequenzselektive Abschirmfunktion, des Gerätes realisiert wird. Z. B. der weiter oben erwähnte Aufbau der Durchführung aus alternierenden LCP- und Metall-Lagen ermöglicht neben der bereits erwähnten Kondensator- sowie Filter-Funktion auch weitere elektrische/elektronische Funktionen speziell in Verbindung mit einem "Daughterboard", das z. B. durch eine flexible Verbindung mit der Durchführung kombiniert ist und direkt im Bereich der Gehäuseöffnung liegt. Bei geeigneter Dimensionierung lassen sich hierdurch insbesondere äußere HF-Störfelder abschirmen und die elektromagnetische Störsicherheit bzw. Verträglichkeit (EMI / EMV) eines entsprechenden Geräts erhöhen.

Die Verbindung der vorgeschlagenen Durchführung mit der Wandung eines Metallgehäuses, speziell Ti-Gehäuses, sollte so gestaltet sein, dass ein Einschweißen in das Gehäuse möglich ist - was eine geeignete Materialauswahl, Geometrie und gegebenenfalls das Vorsehen einer Schweißschutzblende erfordert -, oder die Durchführung wird direkt durch Kleben oder Löten in ein Gehäuseteil (eine Halbschale) eingefügt, insbesondere bevor das Gehäuse zusammengesetzt wird.

Vorteile und Zweckmäßigkeiten der Erfindung ergeben sich im Übrigen aus der nachfolgenden Beschreibung zweier Ausführungsbeispiele anhand der Figuren. Von diesen zeigen:
- Fig. 1A und 1B: eine schematische Längsschnittdarstellung sowie schematische perspektivische Darstellung einer ersten Ausführungsform der Erfindung,
- Fig. 2: eine schematische Längsschnittdarstellung einer zweiten Ausführungsform der Erfindung und
- Fig. 3: eine schematische Längsschnittdarstellung einer dritten Ausführungsform der Erfindung.

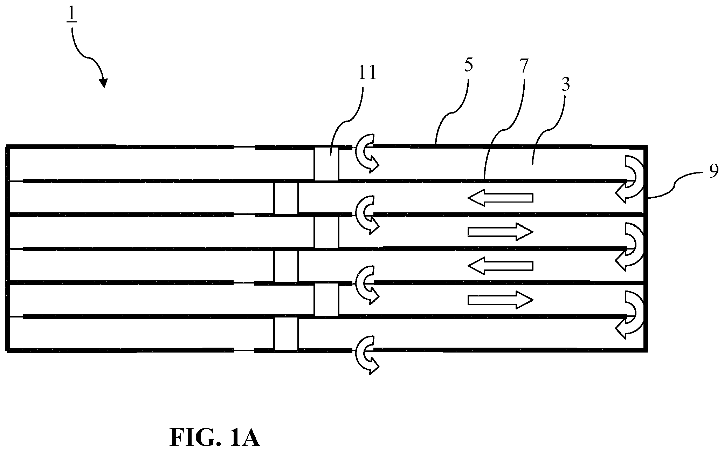

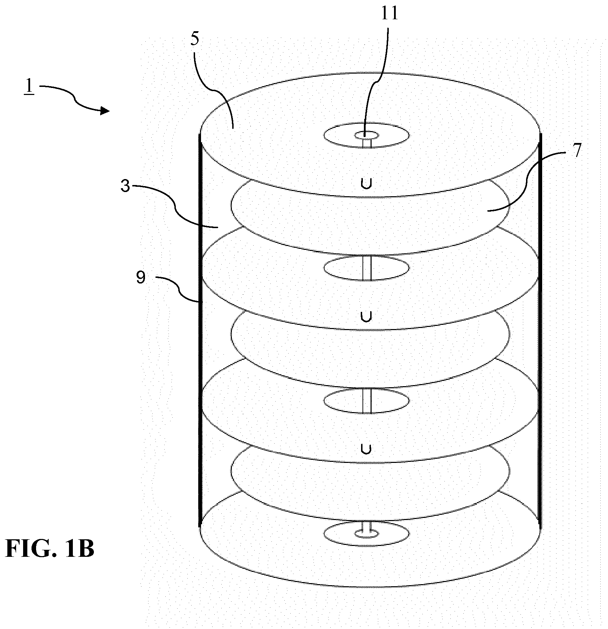

Fig. 1A und 1B zeigen schematisch das Wirkprinzip und den grundsätzlichen Aufbau einer Durchführung 1, die allein aus einem flüssigkristallinen Polymer 3 mit außen angebrachten bzw. eingelagerten ersten und zweiten Metallschichten 5 und 7 aufgebaut ist. Jeweils eine der ersten Metallschichten 5 ist auf den beiden Stirnflächen der im Wesentlichen zylinderförmigen Durchführung 1 angeordnet, und bei der hier dargestellten Ausführung sind zwei weitere der ersten Metallschichten in die Durchführung, parallel zu den Stirnflächen, eingelagert. Die ersten Metallschichten sind jeweils mit einer leitfähigen Mantelschicht 9 der Durchführung kontaktiert und reichen jeweils nicht ganz bis an einen zentralen Leiter (Anschlussleiter) 11 heran. Dem gegenüber sind die jeweils zwischen den ersten Metallschichten 5 in den Durchführungs-Körper eingelagerten zweiten Metallschichten 7 leitend mit dem Anschlussleiter 11 verbunden, reichen aber nicht ganz bis an den Mantel-Leiter 9 heran.

Wie mit den Pfeilen in Fig. 1A symbolisiert, wird dadurch ein labyrinthartiger Aufbau des LCP-Grundkörpers mit erheblich verlängerten Diffusionswegen und verringerten Diffusionsquerschnitten geschaffen.

Der Aufbau nach Fig. 1A und 1B mit den alternierend angeschlossenen Metallschichten 5, 7 bildet einen Kondensator mit den beiden über den leitfähigen Mantel 9 und den Innenleiter 11 realisierten Anschlüssen, dessen Kapazität durch die geeignete Dimensionierung auf einen Wert eingestellt werden kann, der eine Zusatzfunktion im Zusammenwirken mit einer im elektronischen Gerät vorgesehenen Funktionseinheit, etwa eine Ausfilterung äußerer elektromagnetischer Störungen durch ein geeignet abgestimmtes Filter ermöglicht.

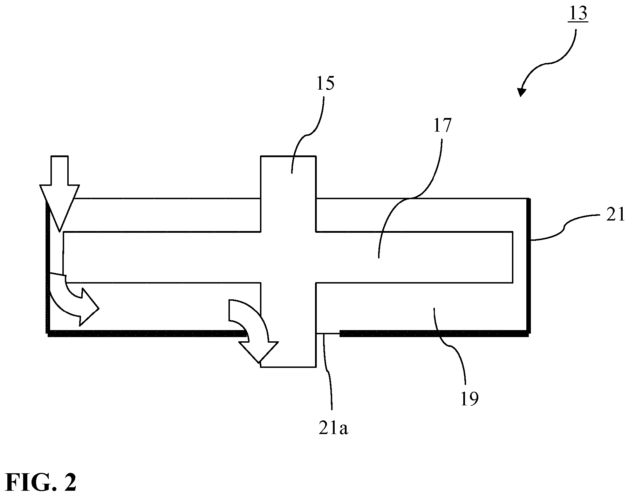

Ein ähnlicher Effekt, wenn auch weniger ausgeprägt, wird mit dem vereinfachten Aufbau einer weiteren Durchführung 13 gemäß Fig. 2 erreicht. Hier ist um einen zentralen Leiter (Anschlussleiter) 15 herum und in elektrischem Kontakt mit diesem eine Metallscheibe 17 angeordnet, die mit einem flüssigkristallinen Polymer 19 derart umspritzt ist, dass die Stirnflächen und der Umfang der Scheibe 17 vollständig in den Polymer eingeschlossen sind. Das hierdurch gewonnene Teil ist in einen Topf 21 aus leitfähigem Material mit einer zentrischen Öffnung 21a eingesetzt, deren Durchmesser größer als derjenige des Anschlussleiters 15 ist. Hierdurch ist für eine elektrische Isolation zwischen Anschlussleiter 15 und Topf 21 gesorgt, und für diffundierende Ionen ergibt sich der wiederum mit Pfeilen bezeichnete, gegenüber einem Durchführungs-Aufbau ohne die Scheibe 17 verlängerte Diffusionsweg und verringerte Diffusionsquerschnitt.

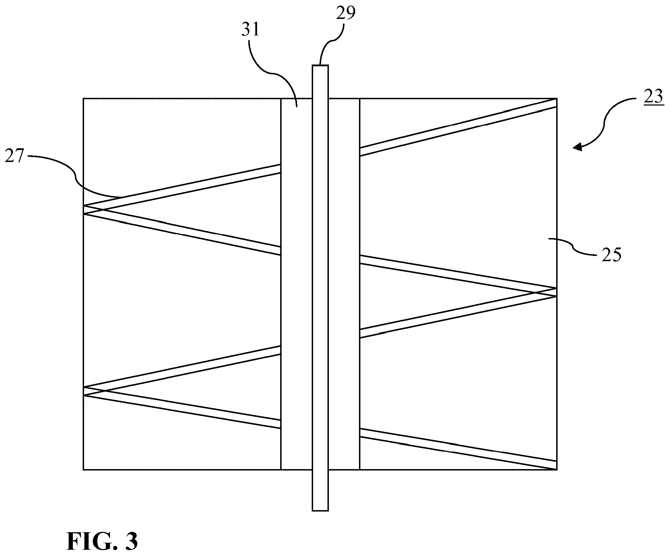

Fig. 3 zeigt schematisch einen weiteren möglichen Aufbau am Beispiel einer Durchführung 23, bei der in einen zylindrischen Grundkörper 25 aus einem flüssigkristallinen Polymer eine spiralig angeordnete Metallfolie 27 eingebettet ist. In einer zentralen Öffnung dieses Durchführungs-Körpers liegt ein Anschlussleiter 29 mit einer Keramikhülle 31. Es handelt sich hier also um einen materialseitig hybriden Durchführungs-Aufbau aus LCP und Keramik.

## Patentansprüche

1. Elektrisches Bauteil mit einem Gehäuse, einer in dem Gehäuse angeordneten Funktionseinheit, einem die Funktionseinheit nach außen elektrisch anschließenden Anschlussleiter (11, 15, 29) und einer dichten, den Anschlussleiter umgebenden und gegenüber dem Gehäuse isolierenden Durchführung (1, 13, 25) im Gehäuse, wobei die Durchführung allein aus einem flüssigkristallinen Polymer (3, 19, 25) gebildet ist, in dem eine oder mehrere Metallschichten (5, 7, 17, 27) eingelagert und/oder an dem eine oder mehrere Metallschichten angebracht sind.

2. Elektrisches Bauteil nach Anspruch 1, ausgebildet als Kondensator, insbesondere Elektrolyt-Kondensator.

3. Elektrisches Bauteil nach Anspruch 1, ausgebildet als Baugruppe einer Nachrichtenübertragungs- oder Filteranordnung.

4. Bauteil nach Anspruch 1, wobei die Durchführung (1) zylinderförmig ist und dadurch eine obere Stirnfläche und eine untere Stirnfläche besitzt und wobei die Durchführung alternierend vom Umfang und von einem Mittenbereich ausgehend verlaufende und jeweils nicht vollständig bis zur Mitte bzw. bis zum Umfang reichende Metallschichten (5, 7) aufweist, die auf den Stirnflächen der Durchführung angeordnet und/oder in dem flüssigkristallinen Polymer innerhalb der Durchführung eingebettet sind und im Längsschnitt eine Labyrinthstruktur bilden, wodurch ein verlängerter Diffusionsweg und ein verringerter Diffusionsquerschnitt für Moleküle bzw. Ionen aus dem Inneren des Geräts nach Außen bestimmt wird.

5. Bauteil nach Anspruch 1, wobei die Durchführung (1) zylinderförmig ist und dadurch eine obere Stirnfläche und eine untere Stirnfläche besitzt und wobei eine spiralig von nahe der oberen Stirnfläche zu nahe der unteren Stirnfläche verlaufende Metallschicht (27) aufweist, die einen verlängerten Diffusionsweg und verringerten Diffusionsquerschnitt für Moleküle bzw. Ionen aus dem Inneren des Gerätes nach Außen bestimmt.

6. Bauteil nach einem der Ansprüche 1 bis 5, wobei die Metallschichten (5, 7, 17, 27) zusammen mit Abschnitten des flüssigkristallinen Polymer (3, 19, 25) eine Kapazität bilden, welche derart angeordnet und an die Funktionseinheit des Gerätes angeschlossen ist, dass hiermit eine elektrische Funktion, insbesondere eine Filterfunktion, des Gerätes realisiert wird.

7. Bauteil nach einem der Ansprüche 1 bis 6, wobei mindestens eine Metallschicht (5, 7, 17, 27), insbesondere eine Mehrzahl von Metallschichten einer Labyrinthstruktur, mit dem Anschlussleiter (11, 15, 29) fest verbunden und/oder integral mit diesem gebildet ist.

## Claims

1. An electronic component, comprising a housing, a functional unit disposed in the housing, a terminal lead (11, 15, 29) electrically connecting the functional unit to the outside, and a sealed feedthrough (1, 13, 25) in the housing, the feedthrough surrounding the terminal lead and insulating it with respect to the housing, wherein the feedthrough is produced substantially solely from the liquid crystal polymer (3, 9, 25), wherein one or more metal layers (5, 7, 17, 27) are incorporated into and/or applied to the liquid crystal polymer .

2. The component according to claim 1, configured as a capacitor, particularly an electrolytic capacitor.

3. The component according to claim 1, configured as an assembly of a message transmission or filtration system.

4. The component according to claim 1, wherein the feedthrough (1) has the shape of a cylinder and thus has an upper end face and a lower end face, wherein the feedthrough comprises alternating metal layers (5, 7) which start at the circumference and a center region and do not fully extend to the center or the circumference and which are disposed on the end faces of the feedthrough and/or embedded in the liquid crystal polymer inside the feedthrough and form a labyrinth structure in the longitudinal section, whereby an elongated diffusion path and reduced diffusion cross-section for molecules and/or ions from the inside of the device to the outside is determined.

5. The component according to claim 1, wherein the feedthrough (1) has the shape of a cylinder and thus has an upper end face and a lower end face, wherein the feedthrough comprises a metal layer (27) extending in a spiral shape from close to the upper end face to close to the lower end face, which determines an elongated diffusion path and a reduced diffusion cross-section for molecules and/or ions from the inside of the device to the outside.

6. The component according to one of claims 1 to 5, wherein the metal layers (5, 7, 17, 27) together with sections of the liquid crystal polymer form a capacitor which is arranged and connected to the functional unit of the device such that in this way an electrical function, in particular a filtration function, of the device is implemented.

7. The component according to one of claims 1 to 6, wherein at least one metal layer (5, 7, 17, 27), in particular a plurality of metal layers of a labyrinth structure, are rigidly connected to the terminal lead (11, 15, 29) and/or integral therewith.

## Revendications

1. Composant électrique pourvu d'un boitier, d'une unité fonctionnelle disposée dans le boitier, d'un conducteur (11, 15, 29) de connexion reliant l'unité fonctionnelle électriquement vers l'extérieur et d'une traversée (1, 13, 25) dans le boitier, étanche, entourant le conducteur de connexion et isolante vis-à-vis du boitier, la traversée seule étant formée à base d'un polymère à cristaux liquides (3, 19, 25) dans lequel une ou plusieurs couches de métal (5, 7, 17, 27) sont incorporées et/ou sur lequel une ou plusieurs couches de métal sont rapportées.

2. Composant électrique selon la revendication 1, conçu sous forme d'un condensateur, notamment un condensateur à électrolyte.

3. Composant électrique selon la revendication 1, conçu sous forme de module d'un agencement de transmission de messages ou d'un agencement de filtrage.

4. Composant électrique selon la revendication 1, dans lequel la traversée (1) est de forme cylindrique et possède ainsi une surface frontale supérieure et une surface frontale inférieure, et dans lequel la traversée présente des couches de métal (5, 7), s'étendant en alternance en partant du périmètre et d'une région centrale et allant non totalement respectivement jusqu'au centre, respectivement jusqu'au périmètre, qui sont disposées sur les surfaces frontales de la traversée, et/ou incorporées dans le polymère à cristaux liquides à l'intérieur de la traversée et forment une structure en labyrinthe dans la section longitudinale, ce par quoi un chemin de diffusion rallongé et une section transversale de diffusion restreinte pour des molécules, respectivement des ions, sont produits à partir de l'intérieur de l'appareil vers l'extérieur.

5. Composant électrique selon la revendication 1, dans lequel la traversée (1) est de forme cylindrique et possède ainsi une surface frontale supérieure et une surface frontale inférieure, et présente ainsi une couche de métal (27) s'étendant en forme de spirale de près de la surface frontale supérieure jusqu'à près de la surface frontale inférieure, qui produit un chemin de diffusion rallongé et une section transversale de diffusion restreinte pour des molécules, respectivement des ions, à partir de l'intérieur de l'appareil vers l' extérieur.

6. Composant électrique selon l'une des revendications 1 à 5, dans lequel les couches de métal (5, 7, 17, 27) forment un condensateur conjointement avec des segments du polymère à cristaux liquides (3, 19, 25), lequel est disposé de telle manière et est raccordé à l'unité fonctionnelle de l'appareil, de sorte qu'ainsi une fonction électrique de l'appareil, notamment une fonction de filtrage, est réalisée.

7. Composant électrique l'une des revendications 1 à 6, dans lequel au moins une couche de métal (5, 7, 17, 27), notamment une multiplicité de couches de métal, d'une structure en labyrinthe est reliée solidement avec le conducteur de connexion (11, 15, 29) et/ou est formée intégralement avec celui-ci.
